# EUROPEAN PATENT APPLICATION

(11) **EP 4 218 726 A1**
(43) Date of publication of application: **02.08.2023**
(21) Application number: 21871689.2
(22) Date of filing: 28.09.2021
(51) Int. Cl.: A61K 9/08, A61K 31/19, A61K 31/045, A61P 11/04, A61P 31/16, A61P 31/02, A61P 31/04, A61P 31/10, A61P 31/12

(54) **COMPOSITION FOR INHIBITING RESPIRATORY VIRUS AND RESPIRATORY VIRUS PREVENTION AND TREATMENT METHOD**

(30) Priority: 28.09.2020 CN 202011040273
(71) Applicant: Shandong University, Jinan, Shandong 250061 (CN)
(72) Inventor: DU, Zongzhan, Jinan, Shandong 250061 (CN); GAO, Qi, Jinan, Shandong 250061 (CN)
(74) Representative: Moré, Solveig Helga
(86) International application number: PCT/CN2021/121378
(87) International publication number: WO 2022/063320

(57) **Abstract**

The present disclosure specifically relates to a composition for inhibiting respiratory viruses and a method for preventing and treating a respiratory virus. Respiratory viral infections are extremely common clinically, yet the current therapeutic drugs for viral infections cannot meet the treatment needs. The present disclosure provides an inhalant for inhibiting respiratory viruses and adopts a method of inhaling high-temperature acetic acid steam for prevention and treatment. Acetic acid in the high-temperature steam can release hydrogen ions to inactivate viruses in the respiratory tract, so as to prevent the onset of the disease and provide a therapeutic effect. In addition, the composition can be used to inhibit fungi and bacteria *in vitro* and prevent skin diseases and respiratory diseases caused by bacteria and fungi. The prevention and treatment method of the present disclosure is easy to implement and has low costs and important clinical application value.

## Description

### TECHNICAL FIELD

The present disclosure belongs to the technical field of biomedicine, and specifically relates to a composition for inhibiting respiratory viruses and a method for preventing and treating a respiratory virus infection.

### BACKGROUND

Disclosure of the related art information is only intended to increase the understanding of the overall background of the present disclosure, and is not necessarily regarded as an acknowledgement or in any form suggesting that the information constitutes the prior art known to those of ordinary skill in the art.

As one of the common clinical diseases, respiratory viral infection is characterized by complex etiology and high viral variability, and is prone to outbreak in the population. At present, there are as many as 100-200 kinds of viruses that can cause respiratory infections, which can be divided into RNA viruses and DNA viruses. The most common pathogenic viruses include influenza viruses, parainfluenza viruses, respiratory syncytial viruses, adenoviruses, rhinoviruses and coronaviruses. In recent years, there have been several new highly pathogenic viruses which mainly infect the respiratory tract, such as SARS coronavirus (SARS-CoV), H5N1 human avian influenza A virus and 2009 novel H1N1 influenza A virus. Due to the aging society, organ transplantation, the application of immunosuppressants in immunity-related diseases, the increase in the incidence of human acquired immunodeficiency syndromes, the increasing number of patients and other factors, the incidence of new or recurring respiratory viral infections is increasing, and the fatality rate of some viral infections is extremely high. Therefore, this type of diseases has become an indispensable public health problem.

Corona Virus Disease 2019 (COVID-19) refers to the pneumonia caused by the 2019 novel coronavirus (2019-nCoV) infection. The structure of the 2019-nCoV includes a protein shell and a single-stranded RNA carrying genetic materials. This simple structure reduces the difficulty of replication of the 2019-nCoV and increases the spread of the virus. The single strand makes the 2019-nCoV more likely to mutate, thereby increasing the difficulty of drug treatment. The main transmission routes of the 2019-nCoV include respiratory droplet transmission, contact transmission, aerosol and fecal-oral transmission. Epidemiological investigations show that people of all ages may be infected, and most victims are adults, among whom the elderly, the weak and the sick seem to be more likely to be infected.

Respiratory viral infections are clinically very common, but there are few corresponding therapeutic drugs. Commonly used clinical antiviral drugs mainly include oseltamivir, amantadines, ribavirin, ganciclovir and Chinese patent medicine containing *Radix scutellariae* or *Andrographis herba*, and the main prevention methods for respiratory viral infections are vaccine prevention, personal hygiene, disinfection of the external environment and the like. The current treatment and prevention drugs cannot fully meet the current needs for the treatment of respiratory viral infections, and the speed of research and development of related drugs is far lower than that of virus transmission and mutation. At present, commonly used disinfection methods include the use of alcohol, 84 disinfectant, etc., but disinfection the external environment of the human body such as the surrounding air, hands and feet cannot kill viruses that have already entered the human respiratory system, and the only thing the infected people can do is to wait for the viruses to replicate and cause diseases.

### SUMMARY

In view of the research background above, an objective of the present disclosure is to provide a method for preventing and treating respiratory virus-related diseases by inactivating respiratory viruses. In order to achieve this objective, the present disclosure provides the following technical schemes:

According to a first aspect of the present disclosure, a composition for inhibiting respiratory viruses is provided, where acetic acid is used as an active ingredient in the composition.

The research of the present disclosure proves that hydrogen ions can inactivate viruses adhering to the human respiratory tract, and acetic acid, as a weak acid, can provide hydrogen ions in a dose that inactivates respiratory viruses at a tolerable concentration, thereby inactivating viruses in the human respiratory tract and preventing and treating respiratory viral infections.

In addition, the composition includes other auxiliary therapeutic ingredients, such as an anti-allergic ingredient, oxygen, ethanol, a fragrance (e.g., citric acid), a colorant, and so onantiallergic. Citric acid and ethanol can not only help relieve the sensory stimulation of the patient caused by acetic acid, but also inactivate viruses and bacteria In addition, when acetic acid is used for treatment, medical oxygen is mixed to increase the blood oxygen concentration of the patient to strengthen the cardiopulmonary function of the patient, thereby improving the effect of inactivating respiratory viruses. The fragrance is conductive to improve the treatment experience of the patient.

According to a second aspect of the present disclosure, an inhalant for inhibiting respiratory viruses is provided, where the inhalant includes the composition according to the first aspect.

The inhalant of the present disclosure also includes a composition scheme containing antiallergic ingredients. Due to individual differences, different people have different tolerances to the ingredients of the above composition. In order to eliminate the possibility of drug allergy and ensure the safety of medication, the inhalant also includes antiallergic ingredients.

According to a third aspect of the present disclosure, a method for preventing and treating respiratory virus infection is provided, including inhalation of the inhalant for inhibiting respiratory viruses according to the second aspect.

In the present disclosure, it is believed through research that in the acetic acid steam at high temperature, acetic acid molecules can ionize to produce more hydrogen ions, which are the main virus inactivating substances. As the temperature of the steam increases, the ionization output is also increased, with the kinetic energy increased substantially. Therefore, increasing the inhalation temperature of the steam as much as possible can further improve the effect of virus inactivation in the respiratory tract.

According to a fourth aspect of the present disclosure, a method for preventing and treating COVID-19 is provided, where the method includes the steps of the method for preventing and treating respiratory virus infection according to the third aspect.

In addition, the method for preventing and treating COVID-19 provided by the present disclosure includes the use of the inhalant in combination with anti-COVID-19 drugs, where α-interferon and the like can be mixed with the inhalant and administered by inhalation; or oral administration or intravenous injection of other anti-COVID-19 drugs is carried out while the inhalant is used for treatment.

According to a fifth aspect of the present disclosure, use of the composition according to the first aspect in inhibiting fungi and/or bacteria *in vitro* is provided.

The composition provided by the present disclosure can also be used in inhibiting fungi and bacteria *in vitro* and in the respiratory tract to prevent fungi or bacteria from adhering to the surface of the skin or respiratory tract to cause diseases. The method for preventing fungi and bacteria in the respiratory tract is the same as that in the third aspect. The method for inhibiting fungi and bacteria on the skin surface includes fumigating the skin surface with the high-temperature steam described in the second aspect.

The beneficial effects of one or more of the above technical schemes are:

The composition and inhalant provided by the present disclosure also have a good effect of killing viruses entering the respiratory system, and can be used for prevention and treatment as well as environmental disinfection. It is proved through experiments that those who use this method for disinfection for about 3 minutes once per day will not be infected with the 2019-nCoV; and those who have been infected with the 2019-nCoV will recover within 4 days by using this method at the early stage.

In addition, the inhalant in the present disclosure has extremely low costs. According to the current price level, each person can be protected from infection with only RMB 0.1 yuan per day, and each patient can be cured with less than RMB 10 yuan.

The inhalation disinfection method in the present disclosure does not have any toxic or side effects on the human body. After killing the viruses, the high-temperature hydrogen ions in the human body can be reduced to normal body temperature in about 0.1 second, form weakly acidic acetic acid with acetate ions, and be excreted with the sputum.

### DETAILED DESCRIPTION OF THE EMBODIMENTS

It should be noted that, the following detailed descriptions are exemplary, and are intended to provide a further description to the present disclosure. Unless otherwise specified, all technical and scientific terms used herein have the same meaning as commonly understood by those of ordinary skill in the art.

It should be noted that terms used herein are only for the purpose of describing embodiments and are not intended to limit the exemplary embodiments of the present disclosure. As used herein, the singular form is intended to include the plural form, unless the context clearly indicates otherwise. In addition, it should further be understood that the terms "comprise" and/or "include" used in this specification indicate that there are features, steps, operations, devices, components, and/or combinations thereof.

As introduced in the background, the current situation is that anti-respiratory virus drugs cannot satisfy clinical applications. In order to solve the above technical problems, the present disclosure provides an inhalant for inhibiting respiratory viruses and a method of treatment.

According to a first aspect of the present disclosure, a composition for inhibiting respiratory viruses is provided, where acetic acid is used as an active ingredient in the composition.

The inhibitory effects on respiratory viruses in the present disclosure includes direct inhibition or killing of viruses, reduction of virus activity, interference with virus adherence, prevention of viruses from entering cells, inhibition of virus transcription and copying processes or inhibition of virus release.

Preferably, the state in which acetic acid inhibits the activity of respiratory viruses is a steam state, specifically, a high-temperature steam state of an acetic acid solution; that is to say, in the composition, the active ingredient for inhibiting respiratory viruses is high-temperature steam of an acetic acid solution.

It has been proved that the composition of the present disclosure is administered by inhalation. Based on this administration method, the above composition can directly act on viruses adhered to the respiratory tract and oral mucosa surface, and has a direct inactivating effect, which is better than the inactivating effecton the viruses in the interior and interlayer of respiratory mucosal cells.

More preferably, the steam acting on viruses is at or above 50°C.

Preferably, the composition also includes one or any mixture of citric acid, ethanol and oxygen.

Further, the oxygen is medical oxygen.

In some embodiments of the present disclosure, the composition includes acetic acid.

In some embodiments of the present disclosure, the composition includes acetic acid and citric acid.

In some embodiments of the present disclosure, the composition includes acetic acid and an anti-allergic agent.

In some embodiments of the present disclosure, the composition includes acetic acid, an anti-allergic agent, and oxygen.

In some embodiments of the present disclosure, the composition includes acetic acid, an anti-allergic agent, oxygen, and a fragrance.

In some embodiments of the present disclosure, the composition includes acetic acid, an anti-allergic agent, oxygen, a fragrance, and a colorant.

In some embodiments of the present disclosure, the composition includes acetic acid and ethanol.

In some embodiments of the present disclosure, the composition includes acetic acid, oxygen and ethanol.

In the above embodiments, a feasible example of the fragrance is citric acid, which is generally used as food preservative and addictive, lemon juice, and fragrances used as food addictive, such as a strawberry flavor, an apple flavor, etc. In an embodiment of the present disclosure, the citric acid can not only help inactivate viruses and bacteria, but also improve the sensory stimulation of the patient caused by the acetic acid. The present disclosure provides a composition with good effects, which is a mixture solution of acetic acid and citric acid including 0.01%-10% by mass of acetic acid and 0.01%-10% by mass of citric acid.

Preferably, the composition further includes an anti-inflammatory drug, an antiviral drug and/or an immunomodulatory drug.

According to a second aspect of the present disclosure, an inhalant for inhibiting respiratory viruses is provided, where the inhalant includes the composition according to the first aspect.

Preferably, the inhalant includes an acetic acid solution with a concentration of 0.01%-10% by mass.

Preferably, the inhalant includes a mixed solution of acetic acid and citric acid. In the mixed solution, the acetic acid has a concentration of 0.01%-10% by mass fraction, and the citric acid has a concentration of 0.01%-10% by mass.

Preferably, the inhalant includes a mixed solution of acetic acid and ethanol. In the mixed solution, the acetic acid has a concentration of 0.01%-10% by mass fraction, and the ethanol has a concentration of 0.01%-10% by mass.

Preferably, the inhalant further includes oxygen.

More preferably, the inhalant includes high-temperature steam of an acetic acid aqueous solution and oxygen.

In some specific embodiments of the above preferred technical schemes, the high-temperature steam of the acetic acid aqueous solution and oxygen in the inhalant are not doped with each other and are inhaled in a certain use order.

In some other embodiments, the steam of the acetic acid aqueous solution and oxygen are mixed in a certain volume ratio and inhaled for use at the same time.

More preferably, the inhalant includes high-temperature steam of an acetic acid and ethanol aqueous solution, and oxygen.

In some specific embodiments of the above preferred technical schemes, the high-temperature steam of the acetic acid and ethanol aqueous solution and oxygen are not doped with each other and are inhaled in a certain use order.

In some other embodiments, the high-temperature steam of the acetic acid and ethanol aqueous solution and oxygen are mixed in a certain volume ratio and inhaled for use at the same time.

Preferably, the inhalant further includes an antiallergic ingredient.

More preferably, the antiallergic ingredients include antihistamine drugs, allergic reaction mediator release-blocking drugs, histamine desensitizers, leukotriene receptor antagonists, drugs for inhibiting antigen-antibody reactions and drugs for alleviating or controlling allergy symptoms.

Further, the antihistamine drugs include but are not limited to diphenhydramine, promethazine and chlorpheniramine.

Further, the allergic reaction mediator release-blocking drugs include but are not limited to sodium cromoglycate and ketotifen.

Further, the histamine desensitizers include but are not limited to betahistine, small-dose histamine diluent and dust mite injection.

Further, the leukotriene receptor antagonists include but are not limited to montelukast and zafirlukast.

Further, the drugs for inhibiting antigen-antibody reactions are adrenal glucocorticoids, immunosuppressants and the like.

Further, the drugs for alleviating or controlling allergy symptoms include but are not limited to smooth muscle antispasmodics such as salbutamol and drugs for reducing edema caused by allergies such as calcium gluconate.

In the above technical schemes regarding the antiallergic ingredients, the antiallergic ingredients are added to the acetic acid and ethanol aqueous solution according to the clinical concentration, where the leukotriene receptor antagonists such as montelukast and zafirlukast are more suitable for respiratory allergies and are added to the inhalant as more preferred ingredients.

In some specific embodiments of the above preferred technical schemes, in the inhalant, the high-temperature steam of an acetic acid, ethanol and antiallergic agent aqueous solution and oxygen are mixed in a certain volume ratio and inhaled for use at the same time.

According to a third aspect of the present disclosure, a method for preventing and treating respiratory virus infection is provided, where the method includes inhalation of the inhalant for inhibiting respiratory viruses according to the second aspect.

Preferably, the respiratory viruses include but are not limited to rhinoviruses, coronaviruses, enteroviruses, adenoviruses and respiratory syncytial viruses.

More preferably, the coronaviruses include but are not limited to SARS-CoV and 2019-nCoV

Preferably, the inhalant has a relatively high temperature that can be tolerated by the human body. It is proved by the present disclosure that the the inhalant is inhaled at or above 50°C.

More preferably, a method of using the inhalant is as follows: heating a part of the inhalant which is in liquid form at room temperature is heated to boil to produce steam, and subjecting a patient to continuous inhalation of the steam for a period of time, where the inhalation temperature of the steam should be maintained at or above 50°C, with the high temperature that the human body can withstand as the upper limit.

In some embodiments of the above preferred technical schemes, the the steam is inhaled for 1-20 min/d.

In some embodiments of the above preferred technical schemes, the concentration of acetic acid in the inhalant is determined according to the tolerance of a patient. It is recommended to use a 0.01%-2% acetic acid solution for children and elderly patients and an acetic acid solution with a concentration of 0.5%-5% for adult patients to achieve the best therapeutic effects.

In some embodiments of the above preferred technical schemes, the inhalant is a mixed aqueous solution of acetic acid and ethanol, where the ratio of acetic acid to ethanol is 1 :0.1-2.

In an embodiment with a good effect of the present disclosure, the inhalant is an acetic acid aqueous solution, the acetic acid aqueous solution is heated to boil, and a patient continuously inhales the steam of the acetic acid aqueous solution at or above 50°C for 1-10 min for treatment.

In an embodiment with a good effect of the present disclosure, the inhalant is an acetic acid and ethanol aqueous solution. The acetic acid and ethanol aqueous solution is heated to boil, and a patient continuously inhales the steam of the acetic acid and ethanol aqueous solution at 50°C or above for 5-10 min for treatment, where the acetic acid and ethanol in the acetic acid and ethanol aqueous solution has a ratio of 1:1 by mass.

In the above series of embodiments, inhalation of oxygen is also included for auxiliary therapy. The oxygen is medical oxygen. The patient inhales the steam and oxygen for a period of time respectively, or mixes the steam and oxygen before inhalation.

Preferably, the prevention and treatment method also includes using other drugs at the same time for treatment. The other drugs include but are not limited to neuraminidase inhibitors, steroidal anti-inflammatory drugs, non-steroidal anti-inflammatory drugs, antibiotics, immunostimulants, immunomodulators, nucleoside antiviral agents, nucleotide antiviral agents, anti-fibrosis drugs, caspase inhibitors, creatinine 5'-monophosphate dehydrogenase inhibitors and viral enzyme inhibitors.

In some specific embodiments, the other drugs include but not limited to chemotherapy drugs, Chinese patent medicine and traditional Chinese medicine, where the traditional Chinese medicine is preferably a decoction, and can be used for fumigation together with the composition.

According to a fourth aspect of the present disclosure, a method for preventing and treating COVID-19 is provided, where the method includes the steps of the method according to the third aspect.

Preferably, the prevention and treatment method is applied to prevention or treatment of COVID-19.

Preferably, the prevention and treatment method also includes administration of anti-COVID-19 drugs in combination. The anti-COVID-19 drugs include but are not limited to α-interferon, lopinavir, ritonavir, ribavirin, favipiravir, chloroquine phosphate or remdesivir.

The 2019-nCoV often enters the host body by adhering to the eyes, nose, mouth, hands and other parts of human body to cause diseases. By using the methods of the present disclosure, the eyes, nose, mouth and other parts which are difficult to disinfect can be effectively disinfected. A user only needs to move his or her face close to the steam of the acetic acid solution and inhale the steam for a period of time to achieve a comprehensive disinfection effect on the eyes, nose and mouth. For users who may be exposed to the 2019-nCoV, inhalation via face mask can also be performed. After mixed with oxygen, the acetic acid steam is introduced into a face mask to disinfect the entire face.

According to a fifth aspect of the present disclosure, use of the composition according to the first aspect in inhibiting fungi and/or bacteria *in vitro* is provided.

Preferably, the part *in vitro* includes the skin surface and the surface of a cavity communicating with the outside; more preferably, the part *in vitro* includes the skin surface and the respiratory tract.

The fungi include common superficial rod shape fungi on the skin surface such as *trichophyton*, *epidermophyton* and *microsporum*, deep infection fungi such as *candidas* and *cryptococcus neoformans*, and other pulmonary system infectious fungi such as *pneumocystis.*

The bacteria are preferably respiratory bacteria, which include but are not limited to one or any mixture of *mycobacterium tuberculosis*, *corynebacterium diphtheria, legionella pneumophila, haemophilus influenzae, bordetella pertussis, mycoplasma pneumoniae* and *chlamydia pneumoniae.*

To make a person skilled in the art understand the technical solutions of the present disclosure more clearly, the technical solutions of the present disclosure are described below with reference to specific examples.

### Example 1

In this example, an inhalant for inhibiting respiratory viruses was provided. The inhalant was a 2.5% acetic acid solution. The inhalant was used as follows: the inhalant was heated to boil, the patient inhaled the steam produced during boiling and fresh air in turn, the inhalation temperature of the steam was kept at or above 50°C, and the patient inhaled the steam at high temperature as far as the patient could tolerate.

The preventive treatment of respiratory virus infection was once a day, and 3 min for each inhalation. The therapeutic treatment of diseases caused by respiratory viruses was three times a day, and 5 min as recommended for each inhalation.

### Example 2

In this example, an inhalant for inhibiting respiratory viruses was provided. The inhalant was a 2% mixed aqueous solution of acetic acid and citric acid (the concentrations of acetic acid and citric acid were both 2%). The inhalant was used as follows: the inhalant was heated to boil, a patient inhaled the steam produced during boiling and fresh air in turn, and the inhalation temperature of the steam should be kept at or above 50°C.

The preventive treatment of respiratory virus infection was once a day, and 3 min for each inhalation. The therapeutic treatment of diseases caused by respiratory viruses was 3-6 times a day, and 5 min as recommended for each inhalation.

### Example 3

In this example, an inhalant for inhibiting respiratory viruses was provided. The inhalant included a 2% mixed aqueous solution of acetic acid and ethanol (the concentrations of acetic acid and ethanol were both 2.5%). The inhalant was used as follows: the inhalant was heated to boil, the patient inhaled a mixture of the steam produced during boiling and the medical oxygen for a period of time, and the inhalation temperature of the steam was kept at or above 50°C.

The preventive treatment of respiratory virus infection was once a day, and 3 min for each inhalation. The therapeutic treatment of diseases caused by respiratory viruses was 3-6 times a day, and 5 min as recommended for each inhalation.

### Example 4

In this example, an inhalant for inhibiting respiratory viruses was provided. The inhalant included medical oxygen and a 2% mixed aqueous solution of acetic acid and ethanol (the concentrations of acetic acid and ethanol were both 2.5%). The inhalant was used as follows: the inhalant was heated to boil, the patient inhaled the mixture of steam produced during boiling and the medical oxygen in turn for a period of time, and the inhalation temperature of the steam was kept at or above 50°C.

The preventive treatment of respiratory virus infection was once a day, and 3 min for each inhalation. The therapeutic treatment of diseases caused by respiratory viruses was 3-6 times a day, and 5 min as recommended for each inhalation.

### Example 5

In this example, an inhalant for prevention and treatment of COVID-19 was provided. The inhalant included medical oxygen and a 2% mixed aqueous solution of acetic acid and ethanol (the concentrations of acetic acid and ethanol were both 2%). The inhalant was used as follows: the inhalant was heated to boil, the patient inhaled the steam produced during boiling and the medical oxygen in turn, and the inhalation temperature of the steam was kept at or above 50°C.

The preventive treatment of COVID-19 was once a day, and 3 min for each inhalation. The therapeutic treatment of COVID-19 was 3-6 times a day, and 5 min as recommended for each inhalation.

### Example 6

In this example, a method for treating COVID-19 was provided. The steps were the same as in Example 4, except that in this example, atomized α-interferon was also inhaled at the same time.

### Example 7

In this example, an inhalant for preventing and treating respiratory bacterial and fungal infections was provided. The inhalant included medical oxygen and a 2.5% mixed aqueous solution of acetic acid, ethanol and montelukast (the concentrations of acetic acid and ethanol were both 2.5%). The inhalant was used as follows: the inhalant was heated to boil, the patient inhaled the steam produced during boiling and the medical oxygen in turn, and the inhalation temperature of the steam was kept at or above 50°C.

The preventive treatment of COVID-19 was once a day, and 3 min for each inhalation. The therapeutic treatment of COVID-19 was 3-6 times a day, and 5 min as recommended for each inhalation.

### Example 8

In this example, an inhalant for preventing and treating respiratory bacterial and fungal infections was provided. The inhalant included medical oxygen, a 2.5% mixed aqueous solution of acetic acid, ethanol and montelukast (the concentrations of acetic acid and ethanol were both 2.5%). The inhalant was used as follows: the inhalant was heated to boil, the patient inhaled the steam produced during boiling and the medical oxygen in turn, and the inhalation temperature of the steam was kept at or above 50°C.

The preventive treatment of COVID-19 was once a day, and 3 min for each inhalation. The therapeutic treatment of COVID-19 was 3-6 times a day, and 5 min as recommended for each inhalation.

### Experimental Example

From 2019 to 2020, 20 influenza patients were recruited as volunteers and the treatment method described in Example 1 was used. A treatment course was 3 days, the cure rate was 100%, and the cure time was one treatment course, where the temperature of patients with high fever can drop to normal level on the second day of treatment.

Wang was a female patient of 40 years old. She had a sudden fever of 39.1 °C at 13 p.m. on June 15, 2020, with headache, sore throat, weakness and slightly stuffy nose, and was diagnosed to have upper respiratory tract infection. She was subjected to the treatment described in Example 1 and inhaled high-temperature acetic acid steam for 3 min every 3 hours. Her temperature decreased to 37.5 °C according to a body temperature measurement at 8 a.m. on June 16, and was 36.8 °C at 8 p.m. on June 16. The symptoms disappeared. A continuous observation of 1 week showed that her body temperature and all other symptoms were normal. The foregoing descriptions are merely preferred embodiments of the present disclosure but are not intended to limit the present disclosure. The present disclosure may include various modifications and changes for a person skilled in the art. Any modification, equivalent replacement, or improvement made within the spirit and principle of the present disclosure shall fall within the protection scope of the present disclosure..

## Claims

1. A composition for inhibiting a respiratory virus, wherein acetic acid is used as an active ingredient in the composition; preferably, the active ingredient for inhibiting respiratory viruses in the composition is high-temperature steam of an acetic acid solution; and more preferably, the steam acting on viruses is at or above 50°C.

2. The composition for inhibiting a respiratory virus according to claim 1, wherein the composition further comprises one or any mixture of ethanol and oxygen; preferably, the oxygen is medical oxygen; further, the composition comprises acetic acid;
or, the composition comprises acetic acid and an antiallergic agent;
or, the composition comprises acetic acid, an antiallergic agent and oxygen;
or, the composition comprises acetic acid, an antiallergic agent, oxygen and a fragrance;
or, the composition comprises acetic acid, an antiallergic agent, oxygen, a fragrance, and a colorant;
or, the composition comprises acetic acid and ethanol;
or, the composition also comprises an anti-inflammatory drug, an antiviral drug and an immunomodulatory drug.

3. An inhalant for inhibiting a respiratory virus, wherein the inhalant comprises the composition according to any one of claims 1-2.

4. The inhalant for inhibiting respiratory viruses according to claim 3, wherein the inhalant comprises an acetic acid solution with a concentration of 0.01%-10%;
or, the inhalant is a mixed solution of acetic acid and ethanol, and in the mixed solution, the acetic acid has a concentration of 0.01%-10%, and the ethanol has a concentration of 0.01%-10%.

5. The inhalant for inhibiting a respiratory virus according to claim 4, wherein the inhalant comprises an acetic acid solution and oxygen;
preferably, the inhalant comprises steam of an acetic acid aqueous solution and oxygen;
further, the steam of the acetic acid aqueous solution and oxygen in the inhalant are not doped with each other and are inhaled in a certain use order; and
further, the steam of the acetic acid aqueous solution and oxygen are mixed in a certain volume ratio and inhaled for use at the same time.

6. The inhalant for inhibiting a respiratory virus according to claim 4, wherein the inhalant also comprises an antiallergic ingredient; preferably, the antiallergic ingredient comprises an antihistamine drug, an allergic reaction mediator release-blocking drug, a histamine desensitizer, a leukotriene receptor antagonist, a drug for inhibiting antigen-antibody reactions and a drug for alleviating or controlling allergy symptoms;
more preferably, the antihistamine drug comprises diphenhydramine, promethazine and chlorpheniramine;
more preferably, the allergic reaction mediator release-blocking drug comprises sodium cromoglycate and ketotifen;
more preferably, the histamine desensitizer comprises betahistine, small-dose histamine diluent and dust mite injection;
more preferably, the leukotriene receptor antagonist comprises montelukast and zafirlukast;
more preferably, the drug for inhibiting antigen-antibody reactions is a adrenal glucocorticoid and an immunosuppressant; and
more preferably, the drug for alleviating or controlling allergy symptoms comprises a smooth muscle antispasmodic such as salbutamol and a drug for reducing edema caused by allergies such as calcium gluconate.

7. A method for preventing and treating respiratory virus infection, wherein the method comprises inhalation of the inhalant for inhibiting respiratory viruses according to any one of claims 3-6;
preferably, the respiratory virus comprises a rhinovirus, a coronavirus, an enterovirus, an adenovirus and a respiratory syncytial virus; further, the coronavirus comprises SARS-CoV and 2019-nCoV;
preferably, the inhalant is inhaled at or above 50°C; further, the inhalant is used as follows: a part of the inhalant which is in liquid form at room temperature is heated to boil to produce steam, a patient continuously inhale the steam for a period of time, and the temperature of the steam is maintained at or above 50°C, with the high temperature that the human body can withstand as the upper limit; further, the steam is inhaled for 1-20 min/d;
further, the concentration of acetic acid in the inhalant is determined according to the tolerance of a patient, a 0.01%-2% acetic acid solution is used for children and elderly patients for treatment, and an acetic acid solution with the concentration of 0.5%-5% is used for adult patients for treatment; and
further, the inhalant is a mixed aqueous solution of acetic acid and ethanol with a ratio of acetic acid to ethanol being 1:0.1-2.

8. The method according to claim 7, wherein the method further comprises using other drugs at the same time for treatment, and the other drugs comprise a neuraminidase inhibitor, a steroidal anti-inflammatory drug, a non-steroidal anti-inflammatory drug, an antibiotic, an immunostimulant, an immunomodulator, a nucleoside antiviral agent, a nucleotide antiviral agent, an anti-fibrosis drug, a caspase inhibitor, a creatinine 5'-monophosphate dehydrogenase inhibitor and a viral enzyme inhibitor;
preferably, the other drugs include but not limited to a chemotherapy drug, Chinese patent medicine and traditional Chinese medicine, where the traditional Chinese medicine is a decoction, and can be used for fumigation together with the composition of claim 1 or 2.

9. A method for preventing and treating Corona Virus Disease 2019 (COVID-19), wherein the method comprises the steps of the method according to claim 7 or 8;
preferably, the method for preventing and treating COVID-19 is applied to prevention or treatment of COVID-19; and
preferably, the method for preventing and treating COVID-19 further comprises administration of an anti-COVID-19 drug in combination, and the anti-COVID-19 drug comprises α-interferon, lopinavir, ritonavir, ribavirin, favipiravir, chloroquine phosphate or remdesivir.

10. Use of the composition according to claim 1 or 2 in inhibiting fungi and/or bacteria *in vitro*, wherein
preferably, an *in vitro* part comprises a skin surface and a surface of a cavity communicating with the outside; more preferably, the in vitro part comprises the skin surface and a respiratory tract;
the fungi comprise *trichophyton, epidermophyton, microsporum, candidas, cryptococcus neoformans* and *pneumocystis*; and
the bacteria are preferably respiratory bacteria comprising one or any mixture of *mycobacterium tuberculosis, corynebacterium diphtheria, legionella pneumophila, haemophilus influenzae, bordetella pertussis, mycoplasma pneumoniae* and *chlamydia pneumoniae.*
